(19) Europäisches Patentamt
European Patent Office
Office européen des brevets

(11) Veröffentlichungsnummer: **0 269 899 B1**

(12) **EUROPÄISCHE PATENTSCHRIFT**

(45) Veröffentlichungstag der Patentschrift: **10.03.93**

(51) Int. Cl.⁵: **A61K 31/19**, A61K 31/235, A61K 31/165

(21) Anmeldenummer: **87116248.3**

(22) Anmeldetag: **04.11.87**

# (54) Verwendung von aromatischen Carbonsäureamiden.

(30) Priorität: **07.11.86 CH 4438/86**

(43) Veröffentlichungstag der Anmeldung:
**08.06.88 Patentblatt 88/23**

(45) Bekanntmachung des Hinweises auf die Patenterteilung:
**10.03.93 Patentblatt 93/10**

(84) Benannte Vertragsstaaten:
**AT BE CH DE FR GB IT LI LU NL SE**

(56) Entgegenhaltungen:
**EP-A- 0 170 105**
**FR-A- 2 226 157**
**US-A- 4 578 498**

**CHEM. PHARM. BULL., 1984, Band 32, Nr. 10, Seiten 4209-4212; H. KAGOCHIKA et al.: "New type inducers of differentiation of human HL-60 promyelocytic leukemia cells. Tereprthalic anilides"**

**PATENT ABSTRACTS OF JAPAN, Band 4, Nr. 54 (C-8)[536], 23. April 1980 & JP-A-5 527 110**

**The Merck Manual, 14 Auflage, 1982, S. 1186-1187 und 2048-2050; Hagers Handbuch der Pharmazeutischen Praxis, 7. Band, Teil A, S. 484-491.**

(73) Patentinhaber: **F. HOFFMANN-LA ROCHE AG**
**Postfach 3255**
**CH-4002 Basel(CH)**

(72) Erfinder: **Klaus, Michael, Dr.**
**Am Hellenrain 6**
**W-7858 Weil/Rhein(DE)**

(74) Vertreter: **Lederer, Franz, Dr. et al**
**Lederer, Keller & Riederer Patentanwälte**
**Lucile-Grahn-Strasse 22**
**W-8000 München 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 269 899 B1

**Beschreibung**

Im Rahmen der vorliegenden Erfingdung wurde festgestellt, dass Verbindungen der Formel

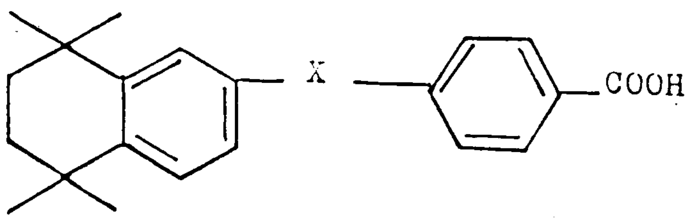

worin X -CONH- oder -NHCO- ist,
und pharmazeutish verwendbare Salze davon zur Behandlung von primärchronischer Polyarthritis geeignet sind.

Die vorliegende Erfindung betrifft somit die Verwendung der Verbindungen der Formel I und pharmazeutisch verwendbarer Salze davon zur Herstellung von Arzneimitteln bei der Behandlung primär-chronischer Polyarthritis.

Beispiele von Salzen der Verbindungen I sind Alkalisalze, wie Natrium-und Kaliumsalze: Erdalkalisalze wie Calciumsalze, und Salze mit organischen Aminen.

Die Verbindungen derFormel I und ihre Salze sind in der europäischen Patentanmeldung A2 - 170 105 beschrieben. Sie werden dort als Wirkstoffe zur Behandlung maligner Erkrankungen, wie Leukämie, cystischer Akne, Psoriasis und verwandten Hautkrankheiten vorgeschlagen. Aus The Merck. Manual, 14., Auflage 1982, S. 1186-1187 und 2048-2050 geht hervor, dass Akne eine inflammatorische Krankheit ist.

Die erfindungsgemässe Verwendung der Verbindungen der Formel I und ihrer Salze erfolgt vorzugsweise in Präparaten zur systemischen Verabreichung.

Die Dosis bei der systemischen Verabreichung variiert gemäss den Bedürfnissen des einzelnen Patienten, wobei eine tägliche Dosis von etwa 5 $\mu$g bis etwa 500 $\mu$g, vorzugsweise etwa 50-100 $\mu$g pro kg Körpergewicht des Patienten in Betracht kommt. Die Dosis kann als Einzeldosis oder auf mehrere Teildosen verteilt verabreicht werden.

Als Verabreichungsform kommen die für die systemische Verabreichung üblichen festen oder flüssigen Darreichungsformen in Betracht, z.B. Suppositorien oder als feste orale Darreichungsformen Kapseln, Tabletten, Dragées, Pillen, Puder, Granulate und dergleichen, als flüssige orale Darreichungsformen Lösungen, Sirupe, Suspensionen, Elixire und dergleichen und als parenterale Darreichungsform Infusions- oder Injektionslösungen, die intravenös oder intramuskulär injiziert werden können. Eine feste Dosierungseinheit, z.B. eine Kapsel, enthält den Wirkstoff vorzugsweise in Mengen von 0,1 mg bis 10 mg.

Die Wirksamkeit der Verbindungen der Formel I im Rahmen der vorliegenden Erfindung kann anhand der nachstehenden Versuchsresultate gezeigt werden:

Gruppen von 10 männlichen Mäusen von einem Minimalgewicht von 20 g wurden am Tage 0 mit methyliertem Rinderserumalbumin (methylated bovine serum albumin; MBSA) sensibilisiert. Die Sensibilisierung erfolgte an zwei Stellen der rasierten Bauchseite durch intradermale Injektion von je 0,05 ml eines 1:1-Gemisches (V/V) von 0,5% MBSA und Freund's vollständigem Adjuvans. 9 Tage später (Tag 8) wurde den Versuchstieren als "Challenge" 0,02 ml 1% MBSA subplantar in eine hintere Pfote injiziert, während in die andere hintere Pfote das gleiche Volumen einer sterilen Kochsalzlösung injiziert wurde. 24 Stunden später (Tag 9) wurde die Entzündung anhand des durch die Injektion verursachten Oedems abgeschätzt. Die Volumina der Pfoten wurden durch Wasserverdrängungs-Plethysmographie gemessen. Die Testverbindung wurde den Versuchstieren an den Tagen 0 bis 4, d.h. während 5 Tagen, oral verabreicht und die Ergebnisse der mit der Verbindung Ia behandelten Tiere mit denjenigen der nur mit dem Vehikel behandelten Kontrolltiere verglichen. Die Resultate wurden wie folgt berechnet: Für jede Maus wurde die prozentuale Zunahme des Pfotenvolumens nach der "Challenge"-Verabreichung des MBSA nach der Formel

$$\frac{\text{"Challenge"-Pfotenvolumen minus Kontrollpfotenvolumen}}{\text{Kontrollpfotenvolumen}} \times 100\%$$

berechnet. Danach wurde die durchschnittliche Zunahme des Pfotenvolumens für jede Gruppe berechnet und die Prozentabnahme des Pfotenvolumens der mit der Testverbindung behandelten Tiere gegenüber den Kontrolltieren wie folgt berechnet:

$$\frac{\text{\% Zunahme des Pfotenvolumens der Kontrolltiere minus \% Zunahme des Pfotenvolumens der behandelten Tiere}}{\text{\% Zunahme des Pfotenvolumens der Kontrolltiere}} \times 100\%$$

Die Resultate sind in Tabelle I zusammengefasst.

Tabelle I

| Verbindung I | Dosis mg/kg/Tag | % Hemmung des Pfotenödems | p < |
|---|---|---|---|
| X = -CONH- | 0,1<br>0,3<br>1,0 | 40<br>63<br>78 | 0,01<br>0,001<br>0,001 |
| X = -NH-CO- | 0,1<br>0,3<br>1,0 | 33<br>46<br>50 | -<br>0,01<br>0,01 |

In ähnlicher Weise wurde die Wirkung im Adjuvans-Arthritis-Test bestimmt, wobei weibliche Ratten (115-170 g) als Versuchstier dienen. Durch Injektion einer Suspension von hitzesterilisierten M. tuberculosis in Paraffinöl in die sub-plantare Oberfläche der rechten Hinterpfote werden primäre Symptome an der Behandlungsstelle und sekundäre Arthritis-Symptome an anderen Körperstellen (nicht-behandelte Pfoten, Nase, Ohr und Schwanz) erzeugt, deren Unterdrückung durch die oral wie oben beschrieben verabreichte Versuchssubstanz vom 7.-17. Versuchstag beobachtet wird (Tabelle II). Von besonderem Interesse ist die markante Unterdrückung der sekundär auftretenden, systemisch verursachten Symptome.

Tabelle II

| Verbindung I | Dosis mg/kg/Tag | % Hemmung des Pfotenödems | | | % Hemmung aller Läsionen |
|---|---|---|---|---|---|
| | | (a) | (b) | (c) | (d) |
| X = -CONH- | 0,03<br>0,1<br>0,3 | 9<br>9<br>28 | 25<br>28<br>50 | 57<br>95<br>98 | 25<br>95<br>98 |
| (a): Pfote mit Injektionsstelle, primäre Läsion (Versuchstag 0-4)<br>(b): Pfote mit Injektionsstelle, sekundäre Läsion (Versuchstag 7-17)<br>(c): Pfote ohne Injektionsstelle, sekundäre Läsion (Versuchstag 7-17)<br>(d): Gesamtbeurteilung (Skala von 0-3) von Nase, Ohren, Pfoten ohne Injektionsstelle, Schwanz | | | | | |

Die Verbindungen der Formel I zeigten eine bemerkenswert niedrige A-Hypervitaminose-Wirkung; Anzeichen einer A-Hypervitaminose (Bollag, Europ. J. Cancer 10, 731 (1974)) wurden erst bei Dosierungen von 3 mg/kg/Tag festgestellt.

Die Herstellung der oben genannten Gebrauchsformen kann in üblicher Weise, z.B. anhand der nachstehenden Beispiele erfolgen.

Beispiel 1

Hartgelatinekapseln enthaltend die folgenden Bestandteile können hergestellt werden:

| Bestandteile | mg/Kapsel | |
|---|---|---|
| | A | B |
| 1. Verbindung I | 0,1 | 10,0 |
| 2. Natriumcarboxymethylcellulose | 9,9 | 10,0 |
| 3. mikrokristalline Cellulose | 281,0 | 271,0 |
| 4. Talk | 8,0 | 8,0 |
| 6. Magnesiumstearat | 1,0 | 1,0 |
| Total | 300,0 | 300,0 |

Verfahren:

Der Wirkstoff wird mit Natriumcarboxymethylcellulose homogen gemischt; diese Mischung wird mit mikrokristalliner Cellulose, Talk und Magnesiumstearat vermischt. Die Endmischung wird in Kapseln der Grösse 0 abgefüllt.

Beispiel 2

Tabletten, enthaltend die folgenden Bestandteile, können wie folgt hergestellt werden:

| Bestandteile | mg/Tablette | |
|---|---|---|
| | A | B |
| 1. Verbindung I | 0,1 | 10,0 |
| 2. Milchzucker pulv. | 130,9 | 121,0 |
| 3. Maisstärke weiss | 30,0 | 30,0 |
| 4. Povidone K30 | 5,0 | 5,0 |
| 6. Maisstärke weiss | 30,0 | 30,0 |
| 7. Magnesiumstearat | 4,0 | 4,0 |
| Total | 200,0 | 200,0 |

Verfahren:

Der feingemahlene Wirkstoff wird mit Milchzucker pulv. und Maisstärke weiss gemischt. Die Mischung wird mit einer wässrigen Lösung von Povidone K30 befeuchtet und geknetet, und die resultierende Masse granuliert, getrocknet und gesiebt. Das Granulat wird mit Maisstärke weiss (2. Teil) und Magnesiumstearat vermischt und zu Tabletten geeigneter Grösse verpresst.

Beispiel 3

Weichgelatinekapseln, enthaltend die folgenden Bestandteile, können wie folgt hergestellt werden:

| Bestandteile | mg/Kapsel |
|---|---|
| 1. Verbindung I | 1,0 |
| 2. Triglycerid | 299,0 |
| Total | 300,0 |

**Patentansprüche**

1. Verwendung von Verbindungen der Formel

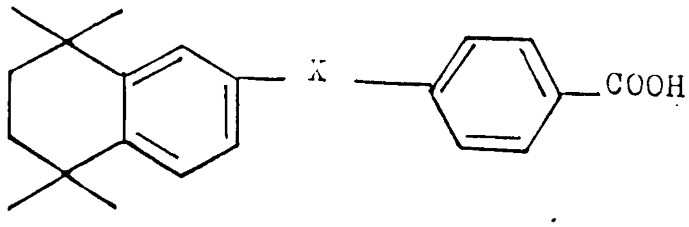

worin X -CONH- oder -NHCO- ist,
und pharmazeutisch verwendbarer Salze davon zur Herstellung von Heilmitteln zur Behandlung von primär-chronischer Polyarthritis.

**Claims**

1. The use of compounds of the formula

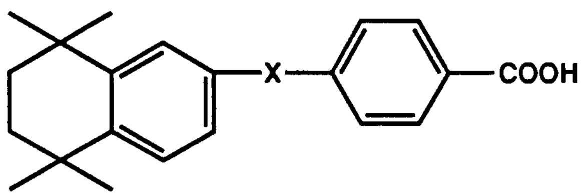

wherein X is -CONH- or -NHCO-,
and pharmaceutically usable salts thereof for the manufacture of medicaments for the treatment of primary chronic polyarthritis.

**Revendications**

1. Utilisation d'un composé de formule

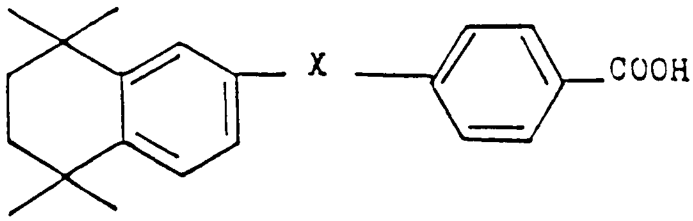

dans lequel X est -CONH- ou -NHCO-, et un sel pharmaceutiquement acceptable pour la préparation d'une composition pharmaceutique pour traiter la polyarthrite chronique primaire.